# EUROPEAN PATENT SPECIFICATION

(11) **EP 3 927 659 B1**
(45) Date of publication and mention of the grant of the patent: **16.10.2024**
(21) Application number: 20705976.7
(22) Date of filing: 20.02.2020
(51) Int. Cl.: C01B 25/222, C01B 25/28, C01B 25/46, C07D 251/56

(54) **PROCESS FOR RECOVERING PHOSPHORIC ACID FROM SOLID PHOSPHORUS SOURCES**
VERFAHREN ZUR RÜCKGEWINNUNG VON PHOSPHORSÄURE AUS FESTEN PHOSPHORQUELLEN
PROCÉDÉ DE RÉCUPÉRATION DE L'ACIDE PHOSPHORIQUE SOLIDE À PARTIR DE SOURCES DE PHOSPHORE SOLIDES

(30) Priority: 21.02.2019 EP 19158557
(43) Date of publication of application: 29.12.2021
(73) Proprietor: Universiteit van Amsterdam, 1012 WX Amsterdam (NL)
(72) Inventor: DE JONG, Gijsbert Bastiaan, 1098 XH Amsterdam (NL); DE BOER, Marissa Adinda, 1098 XH Amsterdam (NL); SLOOTWEG, Jacob Christiaan, 1098 XH Amsterdam (NL)
(74) Representative: De Vries & Metman
(86) International application number: PCT/EP2020/054433
(87) International publication number: WO 2020/169708

(56) References cited:
- GB-A- 1 128 104
- US-A- 2 880 063
- DING Y ET AL: "Technology of preparing ammonium phosphate using solid liquid re-extraction method", WPI / 2017 CLARIVATE ANALYTICS,, vol. 2007, no. 33, 29 November 2006 (2006-11-29), XP002790810
- LI G ET AL: "Forming melamine phosphate used as intermediate for preparing melamine pyrophosphate involves reacting melamine and phosphoric acid in dimethyl sulfoxide, stirring to accelerate reaction at normal temperature, then filtering, and drying", WPI / 2017 CLARIVATE ANALYTICS,, vol. 2014, no. 3, 31 July 2013 (2013-07-31), XP002790811
- DATABASE WPI Week 201403, Derwent World Patents Index; AN 2013-U00396, XP002790811
- DATABASE WPI Week 200733, Derwent World Patents Index; AN 2007-344784, XP002790810

## Description

The invention pertains to a process for preparing phosphoric acid from a solid phosphorus-containing material. The invention also pertains to processes for further converting the phosphoric acid.

Phosphorus is a key element for plant growth and therefore a major element in fertilizer. In consequence, phosphorus deposits around the world are rapidly depleting. There is thus need in the art for a process which can recover phosphorus from waste resources, in particular from solid phosphorus sources. An example of a solid phosphorus source is struvite, which is a solid mineral which can be found, for example, in waste-water treatment units, in waste-water from food processing industries, and in other waste-water streams. Further solid phosphorus-containing waste resources include sewage sludge ash, meat and bone meal ash, and manure ash. Phosphorus can also be recovered from phosphorus-containing minerals such as apatite, vivianite, and phosphate rock in general, where the term phosphate rock is generally used to denote any rock with high phosphorus content.

Methods for recovery of phosphorus in the form of phosphoric acid from solid phosphorus sources are known in the art. In general, they encompass leaching the phosphorus from solid phosphorus sources using an aqueous medium, followed by recovering the phosphorus from the leaching liquid.

For example, US 3298782 describes digestion of phosphate rock with sulphuric acid, followed by extraction of the phosphate using a mixture of water-immiscible alcohol and an amine extractant.

As another example, US 2880063 describes decomposition of a tricalcium phosphate-containing material using aqueous hydrochloric acid, followed by extraction of the phosphoric acid using a water-immiscible solvent.

A problem with methods of this type is the presence of impurities. US 3458282 describes a method for purifying phosphoric acid containing anionic fluoride or sulphate impurities using a specific extractant to extract the impurities form the phosphoric acid.

US 8518359 describes a method for providing phosphoric acid by extracting phosphate ions from solution by absorption on a solid scavenger, and releasing the phosphate into an eluate during regeneration with ammonia, in the form of triammonium phosphate.

GB 1128104 relates to a process for the production of concentrated phosphoric acid. More specifically, it relates to the manufacture of phosphoric acid by the decomposition of rock phosphate with sulfuric acid. Extraction with organic solvent takes place in a separate step. A disadvantage of process described in GB 1128104 is the high temperature used during the decomposition reaction (in excess of 100 °C, usually in excess of 400 °C), at least because hot, concentrated (anhydrous) sulfuric acid is an HSE concern.

It is noted CN 1868865 relates to a process for preparing ammonium phosphate by a solid-liquid back extraction method. This document does not relate to a process for preparing phosphoric acid from a solid phosphorus-containing material.

In addition, it is noted CN 103224471 described a method for the preparation of melamine phosphate. This document also does not relate to a process for preparing phosphoric acid from a solid phosphorus-containing material.

There is need in the art for a process for recovering phosphate from a solid phosphorus-containing material in the form of phosphoric acid which allows the production of phosphoric acid from the solid phosphorus-containing material in high purity, thus with limited co-recovery of contaminants. There is further need for a process which makes efficient use of resources, both of energy and of material. The present invention provides such a process.

The invention is set out in the appended set of claims.

The invention pertains to a process for preparing phosphoric acid from a solid phosphorus-containing material, comprising the steps of
- reacting a solid phosphorus-containing material with strong acid in an amount of 1.0-15 mole acid, calculated as protons, per mole of phosphorus (calculated as P) in the solid phosphorus-containing material in a monophasic reaction medium comprising an organic solvent, to form a solution of phosphoric acid in organic solvent and remaining solid material,
- separating the solution of phosphoric acid in organic solvent from the remaining solid material.

It has been found that phosphoric acid can be recovered from a solid phosphorus-containing material in high purity and efficiency via a solid-state rearrangement/elution process. In other words, it has been found that by carrying out the reaction in a monophasic reaction medium comprising an organic solvent with a specific amount of acid it is possible to selectively form phosphoric acid in solution in the organic solvent, without co-dissolution and extraction of other salts.

Further, the process according to the invention does not require the use of the large amounts of water required by the conventional dissolution/extraction processes known in the art. This makes for a more streamlined process. Additionally, because large volumes of water can be dispensed with, smaller apparatus can be used. The solution of phosphoric acid in organic solvent can be used as a starting material for further processes. Further advantages of the present invention and specific embodiments thereof will become clear from the further specification.

The present invention will be discussed in more detail below.

In the process of the invention a solid phosphorus-containing material is used as starting material. For the process to be economically attractive it is preferred for the solid phosphorus-containing material to contain at least 0.4 wt.% of phosphorus, calculated as P, in particular at least 4 wt.% of phosphorus. If the material is very rich in phosphorus, separation may not be required. Therefore, in general, the amount of phosphorus will be at most 36 wt.%, more specifically at most 32 wt.%.

The solid material is preferably provided in particulate form, that is, in the form of fine particles. It is preferred for the solid material to have a particle size distribution which is such that at least 90 vol.% of the particles have a diameter below 100 micron, in particular below 50 micron. The minimum particle size is not critical, as long as solid-liquid separation can be carried out. As a general indication, it is preferred for at least 90 vol.% of the particles to have a diameter above 0.5 micron.

Examples of suitable materials include struvite, sewage sludge ash, meat and bone meal ash, manure ash, calcium phosphate, phosphate-containing minerals such as apatite, vivianite, and phosphate rock. The present invention is particularly attractive for processing of phosphorus-containing waste materials such as struvite, sewage sludge ash, meat and bone meal ash, calcium phosphate, and manure ash.

The solid material is contacted with a strong acid in an amount of 1.0-15 mole acid, calculated as protons, per mole of phosphorus (P) in the solid phosphorus-containing material. As it is preferred to convert all phosphorus in the solid starting material into phosphoric acid, it is preferred to use at least 3.0 mole acid, calculated as protons, per mole of phosphorus in the solid phosphorus-containing material. A slight excess may be desirable to ensure full conversion, e.g., at least 3.2 mole acid, calculated as protons, per mole of phosphorus in the solid phosphorus-containing material, or at least 3.5 mole/mole. Too much acid should be avoided to prevent dissolution of other materials than phosphoric acid and to prevent the unnecessary use of resources. Therefore, at most 15 mole acid, calculated as protons, is used per mole of phosphorus in the solid phosphorus-containing material. It may be preferred to use at most 12 mole acid, calculated as protons, per mole of phosphorus in the solid phosphorus-containing material, in particular at most 8 mole/mole.

Strong acids within the context of the present specification are acids with a pKₐ below 2, more in particular below 1. Examples of suitable acids are HCl (in gaseous or liquid form), sulphuric acid, nitric acid, trifluoroacetic acid, methanesulphonic acid, and oxalic acid. HCl, nitric acid and sulphuric acid are considered preferred in view of their availability, with sulphuric acid being particularly preferred.

In addition, a mixture of strong and weak acids may also be used to convert the solid phosphorus-containing material into phosphoric acid. Weak acids within the context of the present specification are acids with a pKₐ above 2, more in particular above 2 and below 13. Examples of suitable weak acids are phosphoric acid, acetic acid, citric acid and carbonic acid.

Phosphoric acid may be used in conjunction to a strong acid. Phosphoric acid may react with the phosphorus-containing material leading to a more easily accessible phosphorus material. Therefore, in one embodiment of the invention, a solid phosphorus-containing material is reacted with phosphoric acid followed by or in combination with a strong acid.

In this embodiment, phosphoric acid may be provided in an amount of, e.g., 0.01-5 mole per mole phosphorus (P), in particular in an amount of 0.1-2 mole per mole phosphorus.

Acid can be added in the form of a gas, for HCl, or in liquid form. Where the acid is provided in liquid form, the amount of water in the acid should be monitored to ensure that the reaction medium is still a monophasic reaction medium. The acid may also be provided in the solid form.

The addition of limited amounts of water may help to maximize reaction/elution of phosphoric acid from the solid phosphorus-containing material. Therefore, in one embodiment, the reaction medium may comprise water in addition to the organic solvent.

It is a key feature of the present invention that the reaction medium is monophasic, that is in the form of a single liquid phase, without liquid-liquid phase separation occurring (as can be determined by visual inspection at the reaction temperature). The maximum for the amount of water that may be added to the system is thus governed by the amount of water that can be added before phase separation occurs. This will depend, int. al., on the nature and temperature of the solvent. It is within the scope of the skilled person to determine the maximum amount of water that can be added to the system before phase separation occurs. In general, it is preferred to limit the amount of water, to avoid the leaching of impurities from the solid phosphorus-containing material. Therefore, preferably at most 20 mole of water are used per mole of phosphorus (P) in the phosphorus-containing starting material. In particular, at most 15 mole water is used per mole phosphorus, more in particular at most 10 mole per mole phosphorus.

Where water is present, it is generally present in an amount of at least 0.1 mole per mole phosphorus, e.g., at least 0.2 mole per mole phosphorus.

Water can be added as such, or it can be added in combination with the acid or the organic solvent. The amount of water already present in the solid starting material, organic solvent or other reagents, if any, should also be taken into account.

In the process according to the invention an organic solvent is used. Within the context of the present specification an organic solvent is defined as an organic liquid which has a solubility for phosphoric acid (H₃PO₄) of at least 0.01 mole/liter, determined at 25 ºC. It is preferred for the organic solvent to have a solubility for phosphoric acid of at least 0.05 mole/liter, in particular at least 0.1 mole/liter. Some solvents may have an even higher solubility for phosphoric acid, e.g., at least 0.3 mole/liter, or even at least 0.4 mole/liter. The higher the solubility for phosphoric acid in the organic solvent, the less organic solvent will be required in the process according to the invention. Lower solvent volumes and higher phosphoric acid concentrations in the solvent make for lower reactor volumes and smaller recycle streams. It is within the scope of the skilled person to determine whether an organic liquid meets the required solubility criteria.

Further, the organic solvent has to be liquid under reaction conditions. The reaction is generally carried out at a temperature between -20 ºC and 150 ºC. If a relatively volatile solvent is used, pressure may be applied to keep the reaction medium in the liquid phase.

Examples of suitable solvents are solvents comprising C1-C18 alkyl and alkylene mono- and poly-alcohols, in particular C1-C10 alkyl and alkylene mono- and polyalcohols, more in particular C1-C10 alkylalcohols, e.g., ethanol, propanol, isopropanol, isoamylalcohol (3-methyl-1-butanol).

Other examples of suitable solvents are solvents comprising organophosphate compounds, in particular compounds of the formula R₁R₂R₃PO, wherein R₁, R₂, and R₃ are of the formula R'₁O-, R'₂O-, and R'₃O-, wherein each of R'₁, R'₂, and R'₃ is selected from the group of C1-C10 alkyl and alkylene groups. An examples of a suitable solvent within this group is tri-n-butylphosphate.

It is possible to use mixtures of organic liquids, as long as the liquid mixture acts as an organic solvent as described herein. For example, it is possible to include C8-C16 alkanes into a solvent mixture comprising tri-n-butylphosphate. Tri-n-butylphosphate has a relatively high viscosity, and the inclusion of an organic liquid may make it easier to work with.

The amount of solvent is not critical in the process according to the invention. The lower limit will be determined by the solubility of H₃PO₄ in that sufficient solvent is to be used to take up all phosphoric acid formed. In general, the weight ratio of solvent to solid phosphorus-containing material will be at least 0.1:1, more in particular at least 0.5:1, more in particular at least 1:1, still more in particular at least 2:1, even more in particular at least 5:1. If an excessively large amount of solvent is used, it will only lead to a diluted phosphoric acid solution without additional benefits. Therefore, in general the weight ratio of solvent to solid material will be at most 100:1. A weight ratio in the range of 5:1 to 50:1 may be preferred. Solvent can be added in a single step, or in multiple steps.

Once the solid material, (strong) acid, organic solvent, and water, if necessary, are combined, the thus formed reaction mixture is allowed to react, to allow the formation of phosphoric acid and elution thereof into the organic solvent.

In general, the reaction mixture is allowed to react for at least 2 minutes, in particular at least 5 minutes, more in particular at least 10 minutes, even more in particular at least 1 hour, still more in particular at least 2 hours, most in particular at least 3.5 hours. It may be preferable for the reaction time to be between 10 minutes and 4 hours. At some point in time increasing the reaction time will not lead to further extraction. A very long reaction time, e.g., above 72 hours, will generally not be economically attractive. The suitable reaction time will depend on, e.g., the particle size and nature of the phosphorus-containing material, the amount of acid used, the reaction temperature, the presence or absence of water, and the nature of the solvent. It is within the scope of the skilled person to select an adequate reaction time for the system at issue.

In some embodiments, at least 5% of the phosphorus in the phosphorus-containing starting material, preferably at least 10%, more preferably at least 30%, most preferably at least 70%, is converted to phosphoric acid during the step of reacting a solid phosphorus-containing material with strong acid in a monophasic reaction medium comprising an organic solvent. In general, it is preferred for the conversion to be as high as possible. Therefore, 100% conversion is generally aimed for. As this may require long reaction times, lower degrees of conversion, e.g., at most 90% or at most 80% may be attractive in commercial operation.

The reaction generally takes place at a temperature in the range of -20 ºC to 150 ºC. As temperatures below 0 ºC and very high temperatures may require specific measures, it may be preferred to carry out the reaction at a temperature in the range of 3-80 ºC. As the reaction is exothermic, the temperature of the reaction medium may increase in the first stage of the reaction, and may, depending on the reaction time, decrease when the reaction rate has decreased.

To promote the interaction between the solid material and the liquid components of the reaction medium it is preferred to carry out the reaction under agitation of the reaction medium. This can be carried out by stirring, shaking, or in any other way.

Upon completion of the reaction step, the solution of phosphoric acid in organic solvent is separated from the remaining solid material. This separation step can be carried out via solid-liquid separation steps known in the art, e.g., through filtration, sedimentation, decantation, or through other methods known in the art. The separation results in on the one hand a solution of phosphoric acid in an organic solvent and on the other hand in a solid residue.

Depending on the phosphorus content of the solid residue, it is possible to subject the solid residue to one or more further extraction steps, with organic solvent alone, or with organic solvent, strong acid, and water. The remarks on the various compounds and reaction steps given above also apply to any further reaction steps.

The solution of phosphoric acid in organic solvent obtained in the process according to the invention can be processed as desired.

In one embodiment, phosphoric acid may be recovered from the solvent. This can be effected, e.g., by removal of the solvent through evaporation. It can also be effected by absorption of the phosphoric acid on an absorbent, followed by recovery of the phosphoric acid from the absorbent. The absorbent can, e.g., be an ion exchange resin. If the organic acid has a limited miscibility with water, the phosphoric acid may be removed from the organic liquid through a liquid-liquid extraction step with water.

It is also possible to convert the phosphoric acid into desirable components while still in the organic solvent. In this case, a further reactant is added to the solution of phosphoric acid in organic solvent, wherein the further reactant reacts with phosphoric acid to form a product. In one embodiment the product is in solid form, and can be separated from the organic solvent through solid-liquid separation. In another embodiment, the product is dissolved in the organic solvent. In this case, it can be separated from the organic solvent by methods such as evaporation of the solvent or distillation. In a further embodiment, the product is in the form of a liquid resulting in the formation of a biphasic liquid system. The product and the organic solvent can be separated by liquid-liquid separation.

The organic solvent can then be recycled to the first step of the process according to the invention.

In one embodiment, (inorganic) metal phosphate salts or ammonium salts are formed by adding a base, containing the metal (or metals) of interest as cation(s), or an ammonium base, or ammonia to the solution of phosphoric acid in organic solvent. In the context of the present specification the term metal phosphate also refers to alkaline earth metal phosphate and alkali metal phosphate. Whether the formed salt will be a metal phosphate salt, a metal hydrogen phosphate salt, or a metal dihydrogen phosphate salt will depend on the valence of the metal ion and the ratio between the metal base added and the phosphoric acid. The same applies to ammonium salts, where it will depend on the ratio between the ammonium base and the phosphoric acid whether the formed salt will be a triammonium phosphate salt, a diammonium hydrogen phosphate salt, or a monoammonium dihydrogen phosphate salt. It is within the scope of the skilled person to select a suitable ratio, depending on the desired product. In this context, the term "base" is intended to refer to a hydroxyl-salt, a carbonate salt or a hydride or a hydroxycarbonate salt of the metal or ammonium. The use of hydroxyl-salts is considered preferred. It may be preferred for the base to be added in the form of an aqueous solution, as this may increase the reaction rate with phosphoric acid. An advantage of manufacturing metal or ammonium salts of phosphoric acid from phosphoric acid in an organic solvent is that most salts of phosphoric acid are insoluble in an organic solvent, making for efficient separation of the salt formed from the reaction medium.

The manufacture of salts of alkaline earth metals, in particular of calcium and magnesium phosphate, is considered an attractive embodiment of the process according to the invention. The manufacture of salts of alkaline metals, in particular of sodium and potassium phosphate, is also considered an attractive embodiment of the process according to the invention.

In a preferred embodiment, an ammonium phosphate compound is produced by a process comprising adding ammonia and/or an ammonium base to the solution of phosphoric acid in an organic solvent, resulting in the precipitation of ammonium phosphate compounds, followed by a solid-liquid separation to separate the solid ammonium phosphate compound from the organic solvent. The organic solvent can then be recycled to the first step of the process according to the invention. The ammonium phosphate compound can be washed, and subjected to other purification steps known in the art, such as recrystallization. The ammonium phosphate compound can be mono-ammonium phosphate or diammonium phosphate, depending on the amount of ammonium base added to the system.

The invention thus also pertains to a process for manufacturing an ammonium phosphate compound selected from mono-ammonium phosphate and diammonium phosphate from a solid phosphorus-containing material, which comprises the steps of
- reacting a solid material comprising phosphate salt with strong acid in an amount of 1.0-15 mole acid, calculated as protons, per mole of phosphorus, in the solid phosphorus-containing material in a monophasic reaction medium comprising an organic solvent, to form a solution of phosphoric acid in organic solvent and remaining solid material,
- separating the solution of phosphoric acid in organic solvent from the remaining solid material,
- adding an ammonium base selected from ammonia and ammonium hydroxide to the solution of phosphoric acid in an organic solvent, resulting in the precipitation of ammonium phosphate compounds,
- performing solid-liquid separation to separate the solid ammonium phosphate compound from the organic solvent.

The various preferences expressed above for the process for preparing phosphoric acid from a solid phosphorus-containing material also apply to this embodiment.

In another embodiment of the present invention, an organic amine compound is added to the solution of phosphoric acid in organic solvent, wherein the organic amine compound is selected from the group of primary, secondary, or tertiary amine compounds, which may have one, two, three, or more amine groups. The amine groups react with the phosphoric acid to form ammonium phosphates. In one embodiment the product is in solid form, and can be separated from the organic solvent through solid-liquid separation. The organic solvent can then be recycled to the first step of the process according to the invention.

Where polyamine compounds are used, it will depend on the ratio between the amount of polyamine compound and the amount of phosphoric acid whether mono- or polyphosphate compounds will be formed. It is within the scope of the skilled person to select a suitable ratio, depending on the desired end product.

In a preferred embodiment, melamine phosphate (phosphoric acid; 1,3,5-triazine-2,4,6-triamine) is produced by a process comprising adding melamine, for example in the form of an aqueous solution, to the solution of phosphoric acid in an organic solvent, resulting in the precipitation of melamine phosphate, followed by a solid-liquid separation to separate the solid melamine phosphate from the organic solvent. Depending on the ratio and reaction conditions between melamine and phosphoric acid, the product may be melamine monophosphate, melamine diphosphate, or melamine triphosphate. The term melamine phosphate is intended to encompass all these molecules, and mixtures thereof.

The invention thus also pertains to a process for manufacturing melamine phosphate which comprises the steps of
- reacting a solid material comprising phosphate salt with strong acid in an amount of 1.0-15 mole acid, calculated as protons, per mole of phosphorus, in the solid phosphorus-containing material in a monophasic reaction medium comprising an organic solvent, to form a solution of phosphoric acid in organic solvent and remaining solid material,
- separating the solution of phosphoric acid in organic solvent from the remaining solid material,
- adding melamine to the solution of phosphoric acid in an organic solvent, resulting in the precipitation of melamine phosphate,
- performing a solid-liquid separation to separate the solid melamine phosphate from the organic solvent.

The various preferences expressed above for the process for preparing phosphoric acid from a solid phosphorus-containing material also apply to this embodiment. The melamine may be added in solid form. It may also be added in the form of a solution in water or in an organic medium.

The invention will be elucidated with reference to the following examples, without being limited thereto or thereby.

### Example 1a: Recovery of phosphorus from struvite

9.82 g struvite (40 mmol calculated as phosphorus) was finely ground. 0.62 mL of water was added, as was 50 mL tri-*n*-butylphosphate (TBP) as organic solvent. The mixture was heated to 50 ºC. 3.3 mL of sulphuric acid (95-97%, 60 mmol) was slowly added. In the reaction mixture the molar ratio of water to struvite (calculated as phosphorus) was 1:1. The molar ratio of protons form the sulphuric acid to struvite (calculated as phosphorus) was 3:1.

The reaction mixture was maintained at 50 ºC for 24 hours. The resulting reaction mixture contains solid residue and tri-*n*-butylphosphate as organic solvent. The solid residue was separated from the solvent via filtration. A further 50 mL tri-n-butylphosphate was added to the solid residue, and the mixture was kept at 50 ºC for 24 hours to effect a second extraction. A further filtration step was carried out to separate the solid residue from the solvent.

The liquid filtrate fractions from the two filtration steps were combined to yield 100 mL of tri-n-butylphosphate solvent with a H₃PO₄ concentration of 0.17 M. The total recovery of phosphoric acid was 43%, calculated on the phosphorus in the struvite starting material.

### Example 1b: Production of diammonium phosphate from H₃PO₄ in tri-n-butylphosphate solvent.

A solution of H₃PO₄ in tri-n-butylphosphate was obtained analogous to the method described in example 1a. In contained 100 mL tri-n-butylphosphate with 40 mmol phosphoric acid dissolved therein. The solution was processed as follows. To the solution 7.26 mL NH₄OH (32% w/w in water) was added. A white diammonium phosphate precipitate formed immediately. In the liquid phase, a water phase and a tri-n-butylphosphate phase could be distinguished. 50 mL ethanol was added to create a single phase (as both water and tri-n-butylphosphate are freely miscible with ethanol), and the solid phase was separated from the liquid by filtration. The solid diammonium phosphate residue was washed with 3x 50 mL ethanol. 5.02 g of ammonium phosphate was obtained, which corresponds to a yield of 90.3% (36.1 mmol) analysed with ³¹P{¹H} NMR spectroscopy, calculated as phosphorus in the product as compared to phosphorus in the starting material solution.

### Example 2: Recovery of phosphate from meat and bone meal ash (MBMA) with tri-n-butylphosphate

1.94 g MBMA (5.0 mmol calculated as phosphorus) was finely ground and 0.26 mL water (1.14 mmol) and 10 mL tri-*n*-butylphosphate (TBP) (organic solvent) were added. The mixture was heated to 50 ºC. 0.41 mL of sulphuric acid (95-97%, 7.5 mmol) was added slowly.

The reaction mixture was maintained at 50 ºC for 67 hours. The resulting reaction mixture contained solid residue and tri-n-butylphosphate as organic solvent. The solid residue was separated from the solvent via filtration. A further 10 mL tri-*n*-butylphosphate was added to the solid residue, and the mixture is kept at 50 ºC for 28 hours to effect a second extraction. A further filtration step was carried out to separate the solid residue from the solvent.

The filtrate fractions from the two filtration steps were combined to yield tri-n-butylphosphate solvent with a H₃PO₄ concentration of 0.072 M. The total recovery of phosphoric acid was 28.8% (calculated as phosphorus based on the phosphorus content in the starting material).

### Example 3a: Recovery of phosphorus from struvite with isopropanol

4.91 g struvite (20.0 mmol calculated as phosphorus) was added to a 25 mL round-bottom flask. 0.31 mL H₂O (17.2 mmol) and 25 mL isopropyl alcohol (IPA) as organic solvent, and 1.65 mL H₂SO₄ (97%, 30.0 mmol) was added slowly. The reaction mixture was maintained at room temperature for 21 hours.

The resulting reaction mixture contained solid residue and IPA as organic solvent. The solid residue was separated from the solvent via filtration. A further 25 mL IPA was added to the solid residue, and the mixture was kept at room temperature for 25 hours to effect a second extraction. A further filtration step was carried out to separate the solid residue from the solvent.

The liquid filtrate fractions from the two filtration steps were combined to yield IPA solvent with a total H₃PO₄ concentration of 0.34 M. The total recovery of H₃PO₄ was 84.3% (calculated as phosphorus based on the phosphorus content in the starting material).

### Example 3b: Synthesis of mono-ammonium phosphate

A solution of H₃PO₄ in isopropanol was obtained analogous to the method described in example 3a. The solution contained 16.2 mmol of H₃PO₄ in 45 mL of IPA, to which a molar equivalent of ammonium hydroxide (NH₄OH) (35 w/w%) was added to the solution to generate MAP. Subsequently, 1,8 mL of the NH₄OH solution was added, until no more precipitation was observed. The synthesized MAP was isolated as a colorless solid with a crude yield of 1.73 grams by separating it from the liquid phase by Büchner filtration. Further purification was achieved by recrystallization from 10 mL of boiling deionised water and by adding 20 mL of ethanol, resulting in a colorless crystalline solid that was isolated by filtration. After washing with ethanol and drying (120 °C) to remove any residual ethanol 1.36 grams (11.8 mmol) of MAP was obtained, which corresponds to a yield of 73.1% calculated as phosphorus in the product as compared to phosphorus in the starting material solution. The white crystalline MAP was analysed by melting point analysis, resulting in a melting point of 182.3 °C (literature value: 190 °C (https://pubchem.ncbi.nlm.nih.gov/compound/24402#section=Top, consulted on 25-1-2019).

### Example 3c: Synthesis of melamine phosphate (MP)

A solution of H₃PO₄ in isopropanol was obtained analogous to the method described in example 3a. The solution contained 13.1 mmol of H₃PO₄ in 35 mL of IPA, to which was added a solution of 1.69 grams (13.4 mmol) of melamine (99%) in 9 mL of deionised water. The reaction mixture was heated overnight at 75 °C, which showed an increase in volume of the solid material. The solid material was isolated via Büchner filtration and yielded 2.59 grams (11.6 mmol) of MP which corresponds to 88.5% calculated as phosphorus in the product as compared to phosphorus in the starting material solution. The synthesized MP was analysed by melting point measurement. A slight phase transformation was observed between 290 and 350 °C.

### Example 4: Extraction using various types of solvents

Phosphoric acid extraction from struvite was carried out using different types of solvent. In all experiments 4.91 g struvite (20.0 mmol calculated as phosphorus) was added to a 25 mL RB flask. 0.31 mL H₂O (17.2 mmol) and 25 mL solvent and 1.65 mL H₂SO₄ (97%, 30.0 mmol) were added slowly. The reaction mixture was maintained at a specified temperature for a specified time. The resulting reaction mixture contained solid residue and organic solvent. The solid residue was separated from the solvent via filtration. A further 25 mL of solvent was added to the solid residue, and the mixture was kept at room temperature for 25 hours to effect a second extraction. A further filtration step was carried out to separate the solid residue from the solvent.

The following table (Table 1) shows the solvents used, the time and temperature used in the extraction step, the amount of phosphoric acid obtained in the various steps, and the total recovery.

**Table 1. Recovery of phosphoric acid using various types of solvents**

| solvent | T | 1^{st} extraction | | 2^{nd} extraction | | total extraction | |
|---|---|---|---|---|---|---|---|
| | | time (h) | PA (mole/L) | time (h) | PA (mole/L) | total PA (mole/L) | yield (%) |
| 85% IPE/15%TBP | 20 ºC | 23 | 0.24 | 23 | 0.19 | 0.21 | 52.8 |
| 75% TBP/ 25% DC | 20 ºC | 19 | 0.63 | 27 | 0.071 | 0.35 | 87.5 |
| methanol | 20 ºC | 23 | 0.61 | 30 | 0.007 | 0.31 | 76.5 |
| ethanol | 20 ºC | 21 | 0.62 | 67 | 0.024 | 0.32 | 80.1 |
| ethanol | 50 ºC | 23 | 0.61 | 30 | 0.035 | 0.32 | 80.6 |
| isopropanol | 50 ºC | 21 | 0.44 | 22 | 0 | 0.22 | 54.5 |
| *n*-propanol* | 20 ºC | 24 | 0.33 | - | - | 0.33 | 81.3 |

| | | | | | | | |
|---|---|---|---|---|---|---|---|
| IPE is isopropylether, TBP is tri-n-butylphosphate, DC is dodecane; *40 mmol struvite in 100 mL n-propanol, single extraction. | | | | | | | |

As can be seen from the table, high yield of phosphoric acid can be obtained with different solvents.

### Example 5: Recovery of phosphorus from various solid phosphorus-containing materials

A solid phosphorus-containing material (40 mmol, calculated as phosphorus) was finely ground. A small amount of water (as defined in Table 2) was added. 100 mL n-propanol was added as organic solvent.

When the solid phosphorus-containing material was vivianite, 3.33 mL sulphuric acid (96%, 60 mmol) was slowly added to the mixture of solid phosphorus-containing material, water and n-propanol. The reaction mixture was maintained at 20 °C for 72 h. The resulting reaction mixture contained solid residue and *n*-propanol as organic solvent. The solid residue was separated from the solvent by filtration.

The liquid filtrate fraction comprised n-propanol solvent with H₃PO₄, and was treated with ammonia gas. The resulting reaction mixture contained a solid product and *n*-propanol as organic solvent. The solid product was separated from the solvent *via* filtration.

The total recovery of monoammonium phosphate, calculated based on the phosphorus content of the vivianite starting material, was as indicated in Table 2.

When the solid phosphorus-containing material was sewage sludge ashes (SSA), 6.66 mL sulphuric acid (96%, 120 mmol) was slowly added to the mixture of solid phosphorus material, water and n-propanol. The reaction mixture was maintained at 20 °C for 24 h. The resulting reaction mixture contained solid residue and *n*-propanol as organic solvent. The solid residue was separated from the solvent by filtration.

The liquid filtrate fraction comprised 100 mL *n*-propanol with a H₃PO₄ concentration as defined in Table 2. The total recovery of phosphoric acid, calculated based on the phosphorus content of the phosphorus-containing starting material, was as defined in Table 2.

When the solid phosphorus-containing material was phosphate ore or meat and bonemeal ashes (MBMA), 3.7 mL sulphuric acid (96%, 66.7 mmol) was slowly added to the mixture of solid phosphorus-containing material, water and *n*-propanol. The reaction mixture was maintained at 20 °C for 24 h. The resulting reaction mixture contained solid residue and *n-*propanol as organic solvent. The solid residue was separated from the solvent by filtration. The liquid filtrate fractions comprised 100 mL *n*-propanol with a H₃PO₄ concentration as defined in Table 2. The total recovery of phosphoric acid, calculated based on the phosphorus content of the phosphorus-containing starting material, was as defined in Table 2.

**Table 2: Recovery of phosphorus from various solid phosphorus-containing materials.**

| Solid P-containing material | Vol. H₂O added (mL) | Ratio A | Ratio B | H₃PO₄ conc. (mole/L) | Total recovery |
|---|---|---|---|---|---|
| Vivianite | 4.6 | 6.4:1 | 3:1 | n.a. | 35% |
| Sewage sludge ashes (SSA) | 0.59 | 1:1 | 6:1 | 0.06 | 15.8% |
| Phosphate ore | 2.0 | 3:1 | 3.3:1 | 0.045 | 11.2% |
| Meat and bonemeal ashes (MBMA) | 2.0 | 3:1 | 3.3:1 | 0.05 | 12.6% |

| | | | | | |
|---|---|---|---|---|---|
| Ratio A is amount of water (in moles) per mole phosphorus (P) in the phosphorus-containing starting material in the reaction mixture; ratio B is amount of acid (in moles) per mole of phosphorus in the solid phosphorus-containing material. | | | | | |

### Example 6a: Recovery of phosphorus using various acids

Struvite (9.82 g, 40 mmol calculated as phosphorus) was finely ground. A small amount of water (as defined in Table 3) was added, as was *n*-propanol (100 mL) as organic solvent. An acid (120 mmol) was slowly added. The reaction mixture was maintained at 20 °C for 24 h. The resulting reaction mixture contained solid residue and *n*-propanol as organic solvent. The solid residue was separated from the solvent by filtration.

The liquid filtrate contained 100 mL of n-propanol with a H₃PO₄ concentration as defined in Table 3. The total recovery of phosphoric acid, calculated based on the phosphorus content of the phosphorus-containing starting material, was as defined in Table 3.

**Table 3: Recovery of phosphorus from struvite using various acids**

| Acid | Vol. H₂O added (mL) | Ratio A | Ratio B | H₃PO₄ conc. (mole/L) | Total recovery |
|---|---|---|---|---|---|
| Nitric acid (65% sol.) | 0 | 3.8:1 | 3:1 | 0.21 | 53% |
| Oxalic acid | 0.72 | 1:1 | 6:1 | 0.28 | 70% |
| Methanesulfonic acid (98%) | 0.56 | 1:1 | 3:1 | 0.34 | 84% |

| | | | | | |
|---|---|---|---|---|---|
| Ratio A is amount of water (in moles) per mole phosphorus (P) in the phosphorus-containing starting material in the reaction mixture; ratio B is amount of acid (in moles) per mole of phosphorus in the solid phosphorus-containing material. | | | | | |

### Example 6b: Recovery of phosphorus using hydrochloric acid

Struvite (9.82 g, 40 mmol calculated as phosphorus) was finely ground. Water (0.72 mL) was added, as was 78.4 mL of isopropanol as organic solvent. 21.6 mL of hydrochloric acid in isopropanol (5.51 M, 120 mmol) was slowly added. In the reaction mixture, the molar ratio of water to struvite (calculated as phosphorus) was 1:1. The molar ratio of protons from the hydrochloric acid to struvite (calculated as phosphorus) was 3:1.

The reaction mixture was maintained at 20 °C for 24 h. The resulting reaction mixture contained solid residue and isopropanol as organic solvent. The solid residue was separated from the solvent by filtration.

The liquid filtrate contained 100 mL of isopropanol with a H₃PO₄ concentration of 0.31 M. The total recovery of phosphoric acid was 77%, calculated based on the phosphorus content in the struvite starting material.

### Example 7: Comparison of the process according to the invention and a traditional wet-process

### Example 7a: Process according to the invention

9.82 g struvite (40 mmol, calculated as phosphorus) was finely ground. 0.59 mL water was added, as was 100 mL isoamyl alcohol as organic solvent. 3.33 mL of sulphuric acid (96%, 60 mmol) was slowly added. In the reaction mixture, the molar ratio of water to struvite (calculated as phosphorus) was 1:1. The molar ratio of protons from the sulfuric acid to struvite (calculated as phosphorus) was 3:1.

The reaction mixture was maintained at 20 ºC for 24 hours. The resulting reaction mixture contained solid residue and isoamyl alcohol as organic solvent. The solid residue was separated from the solvent by filtration.

The liquid filtrate fraction contained 100 mL isoamyl alcohol with a H₃PO₄ concentration of 0.14 M. The total recovery of phosphoric acid was 34.2%, calculated based on the phosphorus content in the struvite starting material.

### Comparative example 7b: Traditional wet-process

9.82 g struvite (40 mmol, calculated as phosphorus) was finely ground. 100 mL water was added. 3.33 mL of sulphuric acid (96%, 60 mmol) was slowly added. The molar ratio of protons from the sulphuric acid to struvite (calculated as phosphorus) was 3:1.

The reaction mixture was maintained at 20 ºC for 24 hours. The resulting reaction mixture contained solid residue and water as solvent. The solid residue was separated from the solvent via filtration. The liquid filtrate fraction contained 100 mL of water with a H₃PO₄ concentration of 0.4 M and was extracted with 3 times 100 mL of isoamyl alcohol. The organic solvent fractions were combined and dried with MgSO₄.

The liquid fraction contained 300 mL isoamyl alcohol with a H₃PO₄ concentration 6.4 mM. The total recovery of phosphoric acid was 4.8%, calculated based on the phosphorus content in the struvite starting material.

The process according to the invention (example 7a) thus results in a significantly improved yield, as compared to a traditional wet-process (comparative example 7b). Moreover, in the process according to the invention, no extraction and drying steps are required. Accordingly, the process according to the invention is more efficient and requires less resources, such as solvents and drying materials.

## Claims

1. Process for preparing phosphoric acid from a solid phosphorus-containing material, comprising the steps of
- reacting a solid phosphorus-containing material with strong acid in an amount of 1.0-15 mole acid, calculated as protons, per mole of phosphorus (calculated as P) in the solid phosphorus-containing material in a monophasic reaction medium comprising an organic solvent, to form a solution of phosphoric acid in organic solvent and remaining solid material,
- separating the solution of phosphoric acid in organic solvent from the remaining solid material.

2. Process according to claim 1, wherein the solid phosphorus-containing material contains at least 0.4 wt.% of phosphorus, calculated as P, in particular at least 4 wt.% of phosphorus and/or at most 36 wt.% of phosphorus, calculated as P, more specifically at most 32 wt.%.

3. Process according to any one of the preceding claims, wherein the starting material is selected from the group of struvite, sewage sludge ash, meat and bone meal ash, manure ash, calcium phosphate, phosphate-containing minerals such as apatite, vivianite, and phosphate rock, in particular from the group of struvite, sewage sludge ash, meat and bone meal ash, calcium phosphate, and manure ash.

4. Process according to any one of the preceding claims, wherein the strong acid is used in an amount of at least 3.0 mole acid, calculated as protons, per mole of phosphorus in the solid phosphorus-containing material, in particular at least 3.2 mole/mole, or at least 3.5 mole/mole, and/or at most 12 mole/mole, or at most 8 mole/mole.

5. Process according to any one of the preceding claims, wherein the strong acid is selected from the group of HCl, nitric acid, trifluoroacetic acid, methanesulphonic acid and sulphuric acid, in particular sulphuric acid.

6. Process according to any one of the preceding claims, wherein the reaction medium comprises water in an amount of at most 20 mole water per mole phosphorus (P) in the phosphorus-containing starting material, in particular at most 15 mole water per mole phosphorus (P), more in particular at most 10 mole water per mole phosphorus (P) and/or at least 0.1 mole water per mole phosphorus, e.g., at least 0.2 mole water per mole phosphorus.

7. Process according to any one of the preceding claims, wherein the organic solvent has a solubility for phosphoric acid (H₃PO₄) of at least 0.05 mole/liter, determined at 25 ºC, in particular at least 0.1 mole/liter, or at least 0.3 mole/liter, or even at least 0.4 mole/liter.

8. Process according to any one of the preceding claims, wherein the organic solvent comprises compounds selected from the group of C1-C18 alkyl and alkylene mono- and poly-alcohols and organophosphate compounds, in particular organophosphate compounds of the formula R₁R₂R₃PO, wherein R₁, R₂, and R₃ are of the formula R'₁O-, R'₂O-, and R'₃O-, wherein each of R'₁, R'₂, and R'₃ is selected from the group of C1-C1 0 alkyl and alkylene groups.

9. Process according to any one of the preceding claims, which comprises the further step of recovering phosphoric acid from the organic solvent.

10. Process, which comprises the steps of
preparing a solution of phosphoric acid in organic solvent according to the process of any one of claims 1-8, and
adding a further reactant to the solution of phosphoric acid in organic solvent wherein the further reactant reacts with phosphoric acid to form a product, and separating the product from the reaction medium.

11. Process according to claim 10, wherein phosphate salts of metals, and/or ammonia are formed by adding a base of the metal, an ammonium base, or ammonia to the solution of phosphoric acid in organic solvent, and separating the resulting phosphate salt from the reaction medium.

12. Process according to claim 11, which comprises the step of subjecting the solution of phosphoric acid in the organic solvent to a further reaction to form an ammonium phosphate compound selected from mono-ammonium phosphate and diammonium phosphate, which comprises the steps of adding ammonia or an ammonium base to the solution of phosphoric acid in an organic solvent, resulting in the precipitation of ammonium phosphate compounds, followed by a solid-liquid separation to separate the solid ammonium phosphate compound from the organic solvent.

13. Process, which comprises the steps of
preparing a solution of phosphoric acid in organic solvent according to the process of any one of claims 1-8,
adding an organic amine compound to the solution of phosphoric acid in organic solvent, wherein the organic amine compound is selected from the group of primary, secondary, or tertiary amine compounds, which may have one, two, three, or more amine groups, resulting in the formation of organic ammonium phosphate, and
separating the thus formed organic ammonium phosphate from the organic solvent.

14. Process according to claim 13, which comprises the further step of subjecting the solution of phosphoric acid in the organic solvent to a further reaction to form melamine phosphate, which comprises the steps of adding melamine to the solution of phosphoric acid in an organic solvent, resulting in the precipitation of melamine phosphate, followed by a solid-liquid separation to separate the solid melamine phosphate from the organic solvent.

## Patentansprüche

1. Verfahren zur Herstellung von Phosphorsäure aus einem festen phosphorhaltigen Material, umfassend die Schritte:
- Umsetzen eines festen phosphorhaltigen Materials mit starker Säure in einer Menge von 1,0-15 Mol Säure, berechnet als Protonen, pro Mol Phosphor (berechnet als P) in dem festen phosphorhaltigen Material in einem einphasigen Reaktionsmedium, das ein organisches Lösungsmittel umfasst, um eine Lösung von Phosphorsäure in organischem Lösungsmittel und verbleibendes festes Material zu bilden,
- Abtrennen der Lösung von Phosphorsäure in organischem Lösungsmittel vom verbleibenden festen Material.

2. Verfahren nach Anspruch 1, wobei das feste phosphorhaltige Material mindestens 0,4 Gew.-% Phosphor, berechnet als P, insbesondere mindestens 4 Gew.-% Phosphor und/oder höchstens 36 Gew.-% Phosphor, berechnet als P, insbesondere höchstens 32 Gew.-%, enthält.

3. Verfahren nach einem der vorhergehenden Ansprüche, wobei das Ausgangsmaterial ausgewählt ist aus der Gruppe aus Struvit, Klärschlammasche, Fleisch- und Knochenmehlasche, Gülleasche, Calciumphosphat, phosphathaltigen Mineralien wie Apatit, Vivianit und Phosphatgestein, insbesondere aus der Gruppe aus Struvit, Klärschlammasche, Fleisch- und Knochenmehlasche, Calciumphosphat und Gülleasche.

4. Verfahren nach einem der vorhergehenden Ansprüche, wobei die starke Säure in einer Menge von mindestens 3,0 Mol Säure, berechnet als Protonen, pro Mol Phosphor in dem festen phosphorhaltigen Material verwendet wird, insbesondere mindestens 3,2 Mol/Mol, oder mindestens 3,5 Mol/Mol, und/oder höchstens 12 Mol/Mol, oder höchstens 8 Mol/Mol.

5. Verfahren nach einem der vorhergehenden Ansprüche, wobei die starke Säure ausgewählt ist aus der Gruppe aus HCl, Salpetersäure, Trifluoressigsäure, Methansulfonsäure und Schwefelsäure, insbesondere Schwefelsäure.

6. Verfahren nach einem der vorhergehenden Ansprüche, wobei das Reaktionsmedium Wasser in einer Menge von höchstens 20 Mol Wasser pro Mol Phosphor (P) in dem phosphorhaltigen Ausgangsmaterial enthält, insbesondere höchstens 15 Mol Wasser pro Mol Phosphor (P), insbesondere höchstens 10 Mol Wasser pro Mol Phosphor (P) und/oder mindestens 0,1 Mol Wasser pro Mol Phosphor, z.B. mindestens 0,2 Mol Wasser pro Mol Phosphor.

7. Verfahren nach einem der vorhergehenden Ansprüche, wobei das organische Lösungsmittel eine Löslichkeit von Phosphorsäure (H₃PO₄) von mindestens 0,05 Mol/Liter aufweist, bestimmt bei 25 °C, insbesondere mindestens 0,1 Mol/Liter, oder mindestens 0,3 Mol/Liter, oder sogar mindestens 0,4 Mol/Liter.

8. Verfahren nach einem der vorhergehenden Ansprüche, wobei das organische Lösungsmittel Verbindungen umfasst, ausgewählt aus der Gruppe der C1-C18-Alkyl- und Alkylenmono- und -polyalkohole und Organophosphatverbindungen, insbesondere Organophosphatverbindungen der Formel R₁R₂R₃PO, wobei R₁, R₂ und R₃ die Formeln R'₁O-, R'₂O- und R'₃O- aufweisen,
wobei jedes von R'₁, R'₂ und R'₃ aus der Gruppe der C1-C10-Alkyl- und Alkylengruppen ausgewählt ist.

9. Verfahren nach einem der vorhergehenden Ansprüche, das den weiteren Schritt der Rückgewinnung von Phosphorsäure aus dem organischen Lösungsmittel umfasst.

10. Verfahren, das die Schritte umfasst:
Herstellen einer Lösung von Phosphorsäure in organischem Lösungsmittel gemäß dem Verfahren nach einem der Ansprüche 1 bis 8, und
Zugeben eines weiteren Reaktanten zu der Lösung von Phosphorsäure in organischem Lösungsmittel, wobei der weitere Reaktant mit Phosphorsäure reagiert, um ein Produkt zu bilden, und Abtrennen des Produkts von dem Reaktionsmedium.

11. Verfahren nach Anspruch 10, wobei Phosphatsalze von Metallen und/oder Ammoniak durch Zugabe einer Metallbase, einer Ammoniumbase oder von Ammoniak zu der Lösung von Phosphorsäure in organischem Lösungsmittel und Abtrennen des resultierenden Phosphatsalzes von dem Reaktionsmedium gebildet werden.

12. Verfahren nach Anspruch 11, das den Schritt umfasst: Unterziehen der Lösung von Phosphorsäure in dem organischen Lösungsmittel einer weiteren Reaktion, um eine Ammoniumphosphatverbindung, ausgewählt aus Monoammoniumphosphat und Diammoniumphosphat, zu bilden, was die Schritte des Zugebens von Ammoniak oder einer Ammoniumbase zu der Lösung von Phosphorsäure in einem organischen Lösungsmittel umfasst, was zur Ausfällung von Ammoniumphosphatverbindungen führt, gefolgt von einer Fest-Flüssig-Trennung, um die feste Ammoniumphosphatverbindung von dem organischen Lösungsmittel zu trennen.

13. Verfahren, das die Schritte umfasst:
Herstellen einer Lösung von Phosphorsäure in organischem Lösungsmittel gemäß dem Verfahren nach einem der Ansprüche 1-8,
Zugeben einer organischen Aminverbindung zu der Lösung von Phosphorsäure in organischem Lösungsmittel, wobei die organische Aminverbindung ausgewählt ist aus der Gruppe der primären, sekundären oder tertiären Aminverbindungen, die eine, zwei, drei oder mehr Amingruppen aufweisen können, was zur Bildung von organischem Ammoniumphosphat führt, und
Abtrennen des so gebildeten organischen Ammoniumphosphats von dem organischen Lösungsmittel.

14. Verfahren nach Anspruch 13, das den weiteren Schritt umfasst: Unterziehen der Lösung von Phosphorsäure in dem organischen Lösungsmittel einer weiteren Reaktion, um Melaminphosphat zu bilden, was die Schritte des Zugebens von Melamin zu der Lösung von Phosphorsäure in einem organischen Lösungsmittel umfasst, was zur Ausfällung von Melaminphosphat führt, gefolgt von einer Fest-Flüssig-Trennung, um das feste Melaminphosphat von dem organischen Lösungsmittel zu trennen.

## Revendications

1. Procédé de préparation d'acide phosphorique à partir d'un matériau solide contenant du phosphore, comprenant les étapes suivantes :
- la réaction d'un matériau solide contenant du phosphore avec un acide fort en une quantité de 1,0 à 15 moles d'acide, calculées par rapport aux protons, par mole de phosphore (calculé par rapport à P) dans le matériau solide contenant du phosphore dans un milieu réactionnel monophasique comprenant un solvant organique, pour former une solution d'acide phosphorique dans un solvant organique et un matériau solide restant,
- la séparation de la solution d'acide phosphorique dans un solvant organique à partir du matériau solide restant.

2. Procédé selon la revendication 1, dans lequel le matériau solide contenant du phosphore contient au moins 0,4 % en poids de phosphore, calculé par rapport à P, en particulier au moins 4 % en poids de phosphore et/ou au plus 36 % en poids de phosphore, calculé par rapport à P, plus précisément au plus 32 % en poids.

3. Procédé selon l'une quelconque des revendications précédentes, dans lequel la matière première est sélectionnée dans le groupe comprenant la struvite, les cendres de boues d'épuration, les cendres de farines de viande et d'os, les cendres de fumier, le phosphate de calcium, les minéraux contenant du phosphate tels que l'apatite, la vivianite et le phosphate minéral, en particulier dans le groupe comprenant la struvite, les cendres de boues d'épuration, les cendres de farines de viande et d'os, le phosphate de calcium et les cendres de fumier.

4. Procédé selon l'une quelconque des revendications précédentes, dans lequel l'acide fort est utilisé en une quantité d'au moins 3,0 moles d'acide, calculées par rapport aux protons, par mole de phosphore dans le matériau solide contenant du phosphore, en particulier d'au moins 3,2 moles/mole, ou d'au moins 3,5 moles/mole, et/ou d'au plus 12 moles/mole, ou d'au plus 8 moles/mole.

5. Procédé selon l'une quelconque des revendications précédentes, dans lequel l'acide fort est sélectionné dans le groupe comprenant HCl, l'acide nitrique, l'acide trifluoroacétique, l'acide méthanesulfonique et l'acide sulfurique, en particulier l'acide sulfurique.

6. Procédé selon l'une quelconque des revendications précédentes, dans lequel le milieu réactionnel comprend de l'eau en une quantité d'au plus 20 moles d'eau par mole de phosphore (P) dans la matière première contenant du phosphore, en particulier d'au plus 15 moles d'eau par mole de phosphore (P), plus particulièrement d'au plus 10 moles d'eau par mole de phosphore (P) et/ou d'au moins 0,1 mole d'eau par mole de phosphore, par exemple d'au moins 0,2 mole d'eau par mole de phosphore.

7. Procédé selon l'une quelconque des revendications précédentes, dans lequel le solvant organique présente une solubilité pour l'acide phosphorique (H₃PO₄) d'au moins 0,05 mole/litre, déterminée à 25 °C, en particulier d'au moins 0,1 mole/litre, ou d'au moins 0,3 mole/litre, ou même d'au moins 0,4 mole/litre.

8. Procédé selon l'une quelconque des revendications précédentes, dans lequel le solvant organique comprend des composés sélectionnés dans le groupe comprenant les mono- et poly-alcools d'alkyle et d'alkylène en C1-C18 et les composés d'organophosphate, en particulier les composés d'organophosphate de formule R₁R₂R₃PO, dans laquelle R₁, R₂, et R₃ sont de formule R'₁O-, R'₂O- et R'₃O-, dans lequel chacun parmi R'₁, R'₂ et R'₃ est sélectionné dans le groupe comprenant les groupes alkyle et alkylène en C1-C10.

9. Procédé selon l'une quelconque des revendications précédentes, qui comprend l'étape supplémentaire de récupération d'acide phosphorique à partir du solvant organique.

10. Procédé, qui comprend les étapes suivantes
la préparation d'une solution d'acide phosphorique dans un solvant organique conformément au procédé selon l'une quelconque des revendications 1 à 8, et
l'ajout d'un réactif supplémentaire à la solution d'acide phosphorique dans un solvant organique, dans lequel le réactif supplémentaire réagit avec l'acide phosphorique pour former un produit, et la séparation du produit du milieu réactionnel.

11. Procédé selon la revendication 10, dans lequel des sels de phosphate de métaux, et/ou de l'ammoniac sont formés en ajoutant une base du métal, une base d'ammonium ou de l'ammoniac à la solution d'acide phosphorique dans un solvant organique, et la séparation du sel de phosphate résultant à partir du milieu réactionnel.

12. Procédé selon la revendication 11, qui comprend l'étape de soumission de la solution d'acide phosphorique dans le solvant organique à une réaction supplémentaire pour former un composé de phosphate d'ammonium sélectionné parmi le phosphate de mono-ammonium et le phosphate de diammonium, qui comprend les étapes d'ajout d'ammoniac ou d'une base d'ammonium à la solution d'acide phosphorique dans un solvant organique, ce qui entraîne la précipitation de composés de phosphate d'ammonium, suivie d'une séparation solide-liquide pour séparer le composé de phosphate d'ammonium solide à partir du solvant organique.

13. Procédé, qui comprend les étapes suivantes
la préparation d'une solution d'acide phosphorique dans un solvant organique conformément au procédé selon l'une quelconque des revendications 1 à 8,
l'ajout d'un composé d'amine organique à la solution d'acide phosphorique dans un solvant organique, dans lequel le composé d'amine organique est sélectionné dans le groupe comprenant les composés d'amine primaire, secondaire ou tertiaire, qui peuvent contenir un, deux, trois groupes amine ou plus, ce qui entraîne la formation de phosphate d'ammonium organique, et
la séparation du phosphate d'ammonium organique ainsi formé à partir du solvant organique.

14. Procédé selon la revendication 13, qui comprend l'étape supplémentaire de soumission de la solution d'acide phosphorique dans le solvant organique à une réaction supplémentaire pour former du phosphate de mélamine, qui comprend les étapes d'ajout de mélamine à la solution d'acide phosphorique dans un solvant organique, ce qui entraîne la précipitation du phosphate de mélamine, suivie d'une séparation solide-liquide pour séparer le phosphate de mélamine solide à partir du solvant organique.
